(19)

**Europäisches Patentamt**

**European Patent Office**

**Office européen des brevets**

(11) **EP 3 171 286 A1**

(12) **EUROPEAN PATENT APPLICATION**

(43) Date of publication:
**24.05.2017 Bulletin 2017/21**

(51) Int Cl.:
***G06F 19/00*** (2011.01)

(21) Application number: **15382570.8**

(22) Date of filing: **17.11.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Universitat De València, Estudi General**
  **46010 València (ES)**
• **Universidad Politécnica de Valencia**
  **46022 Valencia (ES)**

(72) Inventors:
• **QUEROL FUENTES, Felipe**
  **46008 VALENCIA (ES)**

• **GARCÍA CASADO, Francisco Javier**
  **46022 VALENCIA (ES)**
• **YE LIN, Yiyao**
  **46022 VALENCIA (ES)**
• **GARCÍA MASSÓ, Xavier**
  **46200 PAIPORTA (ES)**
• **GONZÁLEZ MORENO, Luis Millán**
  **46110 GODELLA (ES)**

(74) Representative: **Juncosa Miro, Jaime**
  **Torner, Juncosa i Associates, S.L.**
  **Gran Via de les Corts**
  **Catalanes, 669 bis, 1o, 2a**
  **08013 Barcelona (ES)**

(54) **METHODS FOR DETERMINING AN IDENTIFIER FOR USE IN METHODS FOR DIAGNOSING HAEMOPHILIC ARTHROPATHY, METHODS AND APPARATUS FOR DIAGNOSING**

(57) Methods for determining a classifier and a classification threshold for use in a method for diagnosing haemophilic arthropathy are disclosed as well as methods for diagnosing haemophilic arthropathy involving such classifiers and classification thresholds. Also computer programs, and computing systems suitable for methods for diagnosing are disclosed.

Fig. 3

**EP 3 171 286 A1**

**Description**

The present disclosure relates to methods for determining classifiers and classification thresholds for use in methods for diagnosing haemophilic arthropathy. It further relates to methods for diagnosing haemophilic arthropathy and to systems, computing apparatus, and computer programs for use in such methods.

BACKGROUND ART

Haemophilia is a group of hereditary genetic disorders that impair the body's ability to control blood clotting or coagulation, which is used to stop bleeding when a blood vessel is broken.

Haemophilia lowers blood plasma clotting factor levels of the coagulation factors needed for a normal clotting process. Thus when a blood vessel is injured, a temporary scab forms, but the missing coagulation factors prevent fibrin formation, which is necessary to maintain the blood clot. A haemophiliac does not bleed more intensely than a person not suffering from haemophilia, but can bleed for a much longer time. In severe haemophiliacs even a minor injury can result in blood loss lasting days or weeks, or even never healing completely.

Characteristic symptoms of haemophelia vary with severity. In general, symptoms are internal or external bleeding episodes, which are called "bleeds". Internal bleeding is common in people with severe haemophilia and some individuals with moderate haemophilia. The most characteristic type of internal bleed is a joint bleed where blood enters into the joint spaces. This is most common with severe haemophiliacs and can occur spontaneously (without evident trauma). If not treated promptly, joint bleeds can lead to permanent joint damage and disfigurement. Bleeding into soft tissues such as muscles and subcutaneous tissues is less severe but can also lead to damage and also requires treatment.

Joint damage from haemarthrosis (i.e. haemophilic arthropathy), potentially with severe pain, disfigurement, and even destruction of the joint and development of debilitating arthritis is a severe complication from haemophilia. The joints that are most commonly affected by haemophilic arthropathy (HA) are the knees, ankles and elbows. Additionally, tissue damage including damage to muscles may occur in the areas surrounding these joints.

Because of the deterioration of the joints, patients suffering from HA generally show poor balance control. In order to properly treat patients, it is important to diagnose HA early and to monitor the progress of the arthropathy correctly. Treatments are generally based on drug administration, but in order to determine the correct doses, proper diagnosis e.g. of the severity of HA is important.

Diagnosis of early stage HA and the progress of HA is nowadays based mostly on physical examination of patients and on the use of medical imaging. Medical imaging requires qualified personnel and costly equipment. A disadvantage of medical imaging for the patient is that he/she can be exposed to several doses of X-rays. On the other hand, physical examination also requires qualified personnel and may be time-consuming for patients. Furthermore, early diagnosis of HA relying on these known techniques may still be relatively complicated.

There thus still exists a need for reliable methods of diagnosing HA which resolves or at least reduces some of the aforementioned problems.

SUMMARY

In accordance with a first aspect, a method for determining a classifier and a classification threshold for use in a method for diagnosing haemophilic arthropathy are provided. The method comprises (i1) a plurality of subjects standing on a pressure platform in a first position during at least a first test period, the pressure platform having a plurality of pressure sensors for measuring a pressure locally; and (ii) receiving data from the pressure sensors indicative of local pressures during the test periods. Then, (iii) based on the received data from the pressure sensors a location of a centre of pressure during the test periods can be determined and (iv) based on the location of the centre of pressure during the test periods (iv1) one or more parameters indicative of a velocity of the centre of pressure during the test periods, (iv2) one or more parameters indicative of a shape of an area covered by the centre of pressure during the test periods, (iv3) one or more parameters indicative of the size of the area covered by the centre of pressure during the test periods.

In accordance with this aspect, the plurality of subjects comprises a first group of subjects and a second group of subjects, wherein the first group of subjects suffer to a different extent from HA than the second group of subjects, and the first position is a unilateral stance on a preferred leg of the subjects and the second position is a unilateral stance on the non-preferred leg of the subjects. (v) The classifier and the classification threshold are determined in a process of machine learning based on the derived parameters for the first and second groups of subjects.

Inventors have found that a classifier and a classification threshold in accordance with this method may be used for making a reliable diagnosis for individual patients in an easy and quick procedure. Once a classifier and classification threshold have been determined, a patient would subsequently only need to perform simple short tests using a pressure platform and then in an automated manner a diagnosis may be made.

Inventors have found that from the movements of the centre of pressure, especially in unilateral stances, information can be derived which allows making a reliable diagnosis of HA. Inventors have further found that particularly the size and shape of the area covered by the centre of pressure, and the velocity during these tests are indicative of the presence or absence of HA. Various different parameters that can be considered indicative of these concepts may be used.

In some examples, valuable information may also be obtained by including test periods in one or more other stances, e.g. a unilateral stance on a non-preferred leg, a bilateral stance with eyes open and a bilateral stance with eyes closed. In some examples, also parameters of a power spectral density (PSD) may be derived during the test periods.

By comparing the parameters for subjects known to suffer from HA with the same parameters for subjects known not to suffer from it, a classifier (and corresponding classification threshold) may be determined through processes of machine learning. Similarly, parameters may be compared for subjects known to have a serious case of HA with subjects known to have a less serious case of HA to obtain another classifier and corresponding classification threshold. In these cases, more than two groups of patients may be defined e.g. groups of subjects may be classified in classes including very severe HA, severe HA, moderate HA, initial stage of HA, no HA. In yet a further alternative, parameters may be compared for subjects known to have HA that is most proliferated in one joint, e.g. knee, with the same parameters for subjects having HA most proliferated in another joint e.g. ankle. Also in this case, more than two groups of patients could be defined.

Machine learning, and in particular supervised learning comprises the task of inferring a function from labelled (i.e. "known") training data. The training data consist of a set of training examples. In supervised learning, each example is a pair consisting of an input object (typically a vector) and a desired output value (also called the supervisory signal). A supervised learning algorithm analyses the training data and produces an inferred function, which can be used for mapping new examples.

In the case at hand, the training data is formed by the parameters determined during the tests of the subjects. The input object in this sense may be regarded as the set of parameters indicative of a velocity, size and shape of the area and a power spectral density and the output object may be regarded as "haemophilic arthropathy" or "not". Or alternatively as "severe HA", "moderate HA" etc. or further alternatively, "HA ankle", "HA knee" etc.

Examples of machine learning that may be employed in examples of the methods of determining classifiers and classification thresholds include Support Vector Machines, logistic regression, naive Bayes, linear or quadratic discriminant analysis, decision trees, k-nearest neighbour algorithm, and Artificial Neural Networks.

Even though various steps and/or processes of the methods have herein been identified with letters and Roman numerals, no specific order is necessarily implied. That is, the order of several method steps may be exchanged without affecting the functionality of the methods.

In another aspect, a method for diagnosing HA is provided. The method comprises (a1) a subject standing on a pressure platform in a first position during at least a first test period, the pressure platform having a plurality of pressure sensors for measuring a pressure locally; and (b) receiving data from the pressure sensors indicative of local pressures during the test periods. (c) Based on the received data from the pressure sensors a location of a centre of pressure during the test periods may be determined. And (d) based on the location of the centre of pressure during the test periods, (d1) one or more parameters indicative of a velocity of the centre of pressure during the test periods, (d2) one or more parameters indicative of a shape of an area covered by the centre of pressure during the test periods, (d3) one or more parameters indicative of the size of the area covered by the centre of pressure during the test periods. The first position is a unilateral stance on a preferred leg of the subject.

Then, (e) a value of a classifier previously derived by a method as substantially hereinbefore described may be calculated. The classifier may be compared with a corresponding classification threshold (f). And based on this determination, the diagnosis of HA may be made.

Even though various steps and/or processes of the methods have been identified with letters, no specific order is necessarily implied. That is, the order of several method steps may be exchanged without affecting the functionality of the methods.

In some embodiments, the steps (b) - (d), preferably the steps (b) - (e) and more preferably the steps (b) - (f) may be implemented in a computing apparatus. In one example, the value of a classifier may be represented to a practitioner on a display. The practitioner may then determine a diagnosis based on the value of the classifier. In another example, the result of the diagnosis is represented on a display to the practitioner. The diagnosis in this sense may be HA (yes or no), degree of HA (moderate, normal, severe), or even indicate the joints likely affected (e.g. knee, ankle). In some cases, a prescription of a pharmaceutical and a dosage regime could be linked directly to the diagnosis.

In some embodiments, the test periods may be between approximately 10 - 40 seconds long, optionally between 15 - 35 seconds long. For the length of the test period various factors may be taken into account. A very short period gives relatively little data for a representative calculation of the different parameters. A very long period gives a large amount of data which requires more time and/or more computational power for processing. Additionally, for unilateral stances, it may not be easy for patients to maintain such a stance for a prolonged period of time.

In yet a further aspect, a computer program product comprising program instructions for causing a computer to perform the steps (b) - (e) or (b) - (f) is disclosed. Such a computer program product may be embodied on a storage medium (for example a CD-ROM, a DVD, a USB drive or a computer memory) or may be carried on a carrier signal (for example, on an electrical or optical carrier signal).

In yet another aspect, a computing system adapted to be connected to a pressure platform having a plurality of sensors and programmed to perform any of the methods according to claims is provided. The computing system may comprise a processor and a memory, with instructions stored in the memory and executable by the processor to execute any of the diagnostic methods substantially ad hereinbefore described.

In yet another aspect, a system for performing a diagnostic method such as substantially hereinbefore described is provided which comprises a pressure platform having a plurality of pressure sensors and a computing system adapted to be connected to the pressure platform.

Additional objects, advantages and features of embodiments of the invention will become apparent to those skilled in the art upon examination of the description, or may be learned by practice of the invention.

BRIEF DESCRIPTION OF THE DRAWINGS

Particular embodiments of the present invention will be described in the following by way of non-limiting examples, with reference to the appended drawings, in which:

Figure 1a schematically illustrates a pressure platform that may be used in examples of methods for determining classifiers and classification thresholds, as well as in examples of methods for diagnosing HA;

Figure 1b schematically illustrates a stabilogram that may result from various tests involving pressure platform;

Figure 2a schematically illustrates an example of a method for determining a classifier and a classification threshold for use in a method for diagnosing HA;

Figure 2b schematically illustrates an alternative example of a method for determining a classifier and a classification threshold; and

Figure 3 schematically illustrates an example of a method for diagnosing HA according to an implementation.

DETAILED DESCRIPTION

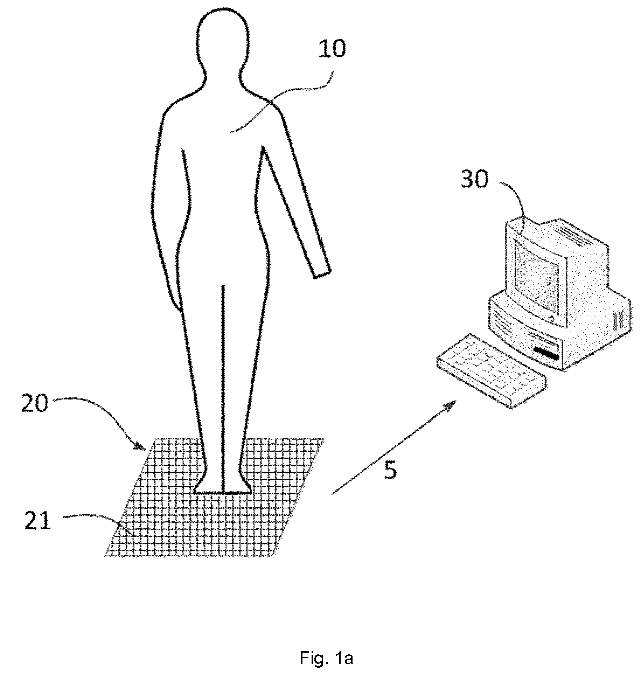

Figure 1a schematically illustrates a pressure platform that may be used in examples of methods for determining classifiers and classification thresholds, as well as in examples of methods for diagnosing HA.

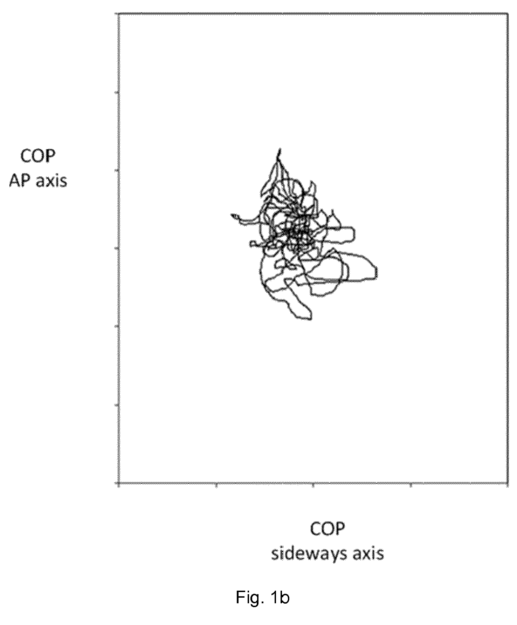

Figure 1 a illustrates a subject 10 standing on a pressure platform 20. Pressure platform 20 may comprise a plurality of pressure sensors. Data from these sensors may be sent 5 to a computing apparatus 30 at a predetermined frequency. Each of the sensors of a pressure platform may indicate the local pressure under the weight of the subject 10. A time series of measured pressures may thus be obtained from each of the sensors.

A subject may assume different stances when standing on a platform. In a first position, the subject may stand in a unilateral stance on his/her preferred leg (right or left), and in a second position, the subject may stand in a unilateral stance on the other leg. Other positions that may be used are a bilateral stance with eyes open and a bilateral stance with eyes closed.

HA is known to affect a person's equilibrium and can thus affect movements of a person during the different tests.

From the data received from sensors 21, a computing apparatus 30 may calculate a center-of-pressure (COP) with a predetermined frequency. A time series indicating the COP during a test period may thus be derived.

Test periods may be between approximately 10 - 40 seconds long, optionally between 15 - 35 seconds long. For unilateral stances, it may not be easy for patients to maintain such a stance for a prolonged period of time. For bilateral stances with eyes closed, as a test progresses, a patient's brain learns how to maintain equilibrium. This may also distort the test data. Inventors have found that good results may be obtained with tests lasting between approximately 10 and 40 seconds, and in particular between 15 and 35 seconds. The test periods do not necessarily need to be of equal length for all the different tests.

Figure 1b schematically illustrates a stabilogram that may result from a test involving a pressure platform 20. Such a stabilogram indicates how a COP may move during such a test. Different stabilograms may be derived from different tests involving unilateral stance on the preferred leg, unilateral stance on a subject's non-preferred leg, and bilateral stances with eyes open and eyes closed.

Such stabilograms may also be decomposed into a movement of the COP along an antero-posterior axis (forward - backward) and a lateral (or "sideways" axis).

From the movements of the COP, different parameters may be derived, including in particular parameters indicative of velocity of movement of the COP during the test periods, parameters indicative of a shape of an area covered by the COP during the test periods, parameters indicative of a size of the area covered by the COP during the test periods and parameters of a power spectral density of the COP during the test periods.

Inventors have found that parameters indicative of these concepts, when combined, have a good "predictive value" for HA, i.e. using a combination of such parameters, a diagnosis of HA may be reliably made.

In the following, different parameters of these different concepts (velocity, shape of area, size of area and PSD) that may be used in various methods according to the present disclosure will be explained in more detail.

Velocity:

Parameters indicative of a velocity of the COP may include one or more of the following:

- Average velocity (MV) along the AP-axis, along the lateral axis and for the resulting position.
- A mean frequency determined as a number of revolutions per second of the COP if the COP had travelled the distance travelled around a circle having a radius equal to the mean distance travelled
- A sum of areas of triangles formed by two consecutive positions of the centre of pressure and an average centre of pressure.

The average velocity may be determined in accordance with Eq. 1:

$$MV = \frac{1}{N-1} \sum_{i=1}^{N-1} v(i) \qquad \text{Eq. 1}$$

, wherein

MV is the mean velocity , and v(i) may be determined in accordance with Eq. 2:

$$v(n) = \frac{|RD(n+1) - RD(n)|}{T} \qquad \text{Eq. 2}$$

, wherein

V(n) is the instantaneous velocity at any given moment, RD is the signal of the displacement of the COP (with respect to an origin), N is the total number of samples, and T is the sampling period (1/f, wherein f is the frequency with which data is received from the sensors of the pressure platform).

RD is the resultant distance from the displacement along the antero-posterior axis (AP) and the displacement along the midway-lateral axis (ML). For this reason:

$$RD = \sqrt{AP^2 + ML^2} \qquad \text{Eq. 3}$$

The average velocity in accordance with these equations may be calculated for the total movement (RD), for the movement along the antero-posterior axis (AP) and for the movement along the lateral axis (ML).

For example, the

$$v_{AP}(n) = \frac{|AP(n+1) - AP(n)|}{T} \qquad \text{Eq. 2a}$$

Total displacement (TD) may be calculated by summing up all the distances between consecutive samples:

$$TD = \sum_{i=1}^{N-1} \left| RD(n+1) - RD(n) \right|$$ Eq. 4

Similarly, the total distance along each of the axes may be determined in accordance with:

$$TD_{AP} = \sum_{i=1}^{N-1} \left| AP(n+1) - AP(n) \right|$$ , and Eq. 4a

$$TD_L = \sum_{i=1}^{N-1} \left| ML(n+1) - ML(n) \right|$$ Eq. 4b

The mean distance of the COP may be determined in accordance with:

$$MDIST = \frac{TD}{N}$$ Eq. 5

The mean distance of the COP may also be calculated along each of the axes anterio-posterior and lateral:

$$MDIST_{AP} = \frac{TD_{AP}}{N},$$ Eq. 5a

and $$MDIST_L = \frac{TD_L}{N}$$ Eq. 5b.

From these equations, the mean frequency determined as a number of revolutions per second of the COP if the COP had travelled the distance travelled around a circle having a radius equal to the mean distance travelled as defined before may be determined in accordance with:

$$MFREQ = \frac{TD}{2\pi MDIST.T}$$ Eq. 6

The mean frequency may also be determined in a similar manner along the AP axis and the ML axis.
A sum of areas of triangles formed by two consecutive positions of the centre of pressure and an average centre of pressure ("Sway area (AREA-SW)") may be calculated in accordance with:

$$AREA - SW = \frac{1}{2T} \sum_{n=1}^{N-1} \left| AP[n+1]ML[n] - AP[n]ML[n+1] \right|$$ Eq. 7

Power Spectral Density

Parameters of the Power Spectral Density (PSD) of the COP may include one or more of the following:

- a total power of the power spectral density,
- a power in low frequencies between 0.015 and 0.5 Hz, (which is an indicator of the implication of the visual and vestibular system in maintaining equilibrium)
- a power in median frequencies between 0.5 - 2 Hz, (which is an indicator of the implication of the cerebellum in maintaining equilibrium)
- a power in high frequencies between 2 - 6 Hz, (which is an indicator of the implication of the proprioceptive system in maintaining equilibrium)
- a median power frequency being equal to a frequency below which 50% of the total power lies,
- a 95% power frequency being equal to a frequency below which 95% of the total power lies,
- a centroidal frequency being a frequency at which the spectral mass is concentrated, and
- a frequency dispersion being a measure of a variability of the frequency content of the power spectral density.

These parameters are all parameters in the frequency domain.

From the COP signals (resultant distance RD, and positions along the antero-posterior axis and lateral axis), also the power spectral densities may be determined from the power spectrum of the signal, $G_{RD}(f)$, $G_{AP}(f)$ and $G_{ML}(f)$, which may be estimated through a Fourier transformation.

Let f be the frequency vector of Gm, and $\Delta f$ the frequency increment in the discrete power spectral density estimate G[m]. Spectral moments may be defined in accordance with:

$$\mu_k = \sum_{m=i}^{j} \left( m\Delta f \right)^k G[m]$$

Eq. 8

Total power of the total power spectral density is a mean square value of the time series:

$$POWER = \mu_0 = \sum_{i=1}^{N} G_m(i)$$

Eq. 9

Similar equations may be derived for the power of the PSD at low frequencies (0.015 - 0.5 Hz), medium frequencies (0.5 - 2 Hz) and high frequencies (2 - 6 Hz).

The 50% power frequency, or median power frequency is $u\Delta f$, wherein u is the smallest integer for which:

$$\sum_{m=1}^{u} G(m) \geq 0.50\,\mu_0$$

Eq. 10

The 95% power frequency, the frequency below which 95% of the total power is found is $v\Delta f$, where v is the smallest integer for which:

$$\sum_{m=i}^{v} G(m) \geq 0.95\,\mu_0$$

Eq. 11

The centroidal frequency is the frequency at which the spectral mass is concentrated, and may be calculated in accordance with:

$$CFREQ = \left[ \frac{\mu_2}{\mu_0} \right]^{\frac{1}{2}}$$

Eq. 12

The frequency dispersion is a unitless measure of the variability in the frequency content of the power spectral density and may be calculated in accordance with:

$$FREQD = \left[ 1 - \frac{\mu_1^2}{\mu_0 \mu_2} \right]^{\frac{1}{2}}$$

Eq. 13

Size of area

Parameters indicative of the size of the area covered by the centre of pressure may include one or more of the following:

- an area of a circle encompassing the centre of pressure 95% of the time,
- an area of an ellipse encompassing the centre of pressure 95% of the time,
- a sum of areas of triangles formed by two consecutive positions of the centre of pressure and an average centre of pressure.

An area of a circle encompassing the centre of pressure 95% of the time may be calculated in accordance with:

$$AREA\_CC = \pi \left( MDIST + z_{0,5} SD_{RD} \right)^2 ,$$

Eq. 14

wherein $z_{0,5}$ is the z-statistic at 95% confidence level, namely 1.645 and $SD_{RD}$ is the standard deviation of the time series of the distance and may be calculated in accordance with:

$$SD_{RD} = \left[ 1/N \sum RD^2(n) - MDIST^2 \right]^{\frac{1}{2}} = \left[ RMS^2 - MDIST^2 \right]^{\frac{1}{2}}$$
(Eq. 15)

, wherein

The Root Mean Square (RMS) of the positions of the COP may be calculated in accordance with:

$$RMS = \sqrt{ \frac{\sum_{i=1}^{N} (RD(i))^2}{N} }$$

Eq. 16

Similarly, along e.g. the AP-axis, the root mean square may be determined with:

$$RMS_{AP} = \sqrt{\frac{\sum_{i=1}^{N}(AP(i))^2}{N}} \qquad\qquad \text{Eq. 16a}$$

And it will be clear that a similar equation may be used for the root mean square along the lateral axis.

An area of an ellipse encompassing the centre of pressure 95% of the time may be calculated in accordance with:

$$AREA\_CE = \pi\left(maj_{axis}.\min_{axis}\right) \qquad\qquad \text{Eq. 17,}$$

wherein

$$maj_{axis} = \sqrt{2.\left(SD_{AP}^2 + SD_{ML}^2 + D\right)} \qquad \text{, and} \qquad \text{Eq. 17a}$$

$$\min_{axis} = \sqrt{2.\left(SD_{AP}^2 + SD_{ML}^2 - D\right)}, \qquad\qquad \text{Eq. 17b,}$$

wherein

$$D = \sqrt{\left(SD_{AP}^2 + SD_{ML}^2\right) - 4.\left(SD_{AP}^2.SD_{ML}^2 - S_{TD}^2\right)} \text{, and} \qquad \text{Eq. 18}$$

$$S_{APML} = \frac{1}{N}\sum_{i=1}^{N} AP(i).ML(i), \qquad\qquad \text{Eq. 19}$$

wherein $S_{APML}$ is the covariance between the displacement of the COP in the AP and ML axes.

<u>Shape of area</u>

Parameters indicative of a shape of an area covered by the centre of pressure may include one or more fractal dimensions indicative of the degree to which the position of the centre of pressure fills a metric space which it encompasses, including one or more of the following:

- a fractal dimension wherein the metric space is a circle having a diameter which is equal to the range of the centre of pressure, and/or
- a fractal dimension wherein the metric space is a circle having a radius such that the centre of pressure is within the circle 95% of the time and/or
- a fractal dimension wherein the metric space is an ellipse such that the centre of pressure is within the ellipse 95% of the time

The fractal dimension (FD) is a unitless measure of the degree to which a curve fills a metric space which it encompasses.

$$FD = \log(n) / \log(n \cdot d / TD) \qquad\qquad \text{Eq. 20}$$

, wherein d represents a planar diameter of the curve, or the maximum distance between any two points. The fractal dimension wherein the metric space is a circle having a diameter which is equal to the range of the COP may be determined in accordance with Eq 20.

Fractal dimension-CC is based on the 95% confidence circle according to equations 14 and 15 and may be found by substituting "d" in equation 20 by.

$$ d_{FD-CC} = 2\left(MDIST + z_{.05 S_{RD}}\right) \qquad \text{Eq. 21} $$

Fractal dimensions-CE models the area of the stabilogram with the 95% confidence ellipse as derived from equations 17 - 19. This fractal diemsnions may be found by substituting "d" in equation 20 by:

$$ d_{FD-CE} = \sqrt{(2a2b)} \qquad \text{Eq. 22} $$

Other parameters that may be taken into account include the range (total displacement of the centre of pressure) and one or more parameters derived from a stabilogram diffusion analysis, the Hurst exponent of the COP along each of the axes (RD, AP and ML).

The range may be calculated in accordance with:

$$ Range = \max(RD) - \min(RD) \qquad \text{Eq. 23} $$

Again, similar equations can be written for the range along the antero-posterior axis and the lateral axis.

Parameters derived from a stabilogram diffusion analysis and an R/S analysis may include:

- Short term diffusion coefficient
- Long term diffusion coefficient
- Short term Hurst exponent
- Long term Hurst exponent
- Critical point (point of change from short terms to long term)

A starting point is

$$ \left\langle \Delta RD^2 \right\rangle_m = \frac{1}{N-m} \sum_{i=1}^{N-m} \left(RD(i+m) - RD(i)\right)^2 \qquad \text{Eq. 24} $$

These data are plotted on a graph of $\Delta t$ versus $\Delta RD^2$ on a logarithmic scale. On this graph the slope of the short-term and long-term and where the turning point is located can be determined.

The Hurst exponent may be calculated in accordance with the following procedure and equations:

$$ y(n) = \sum_{i=1}^{n} \left(RD(i) - \overline{RD_\tau}\right) \qquad \text{Eq. 25} $$

Then, the following equations can be calculated:

$$ R(\tau) = \max(y) - \min(y) \qquad \text{Eq. 26} $$

$$S(\tau) = \sqrt{\frac{1}{\tau} \sum_{i=1}^{\tau} (RD(i) - \overline{RD\tau})^2}$$

Eq. 27

The logarithm of (R / S) can then be plotted vs the logarithm of $\tau$ and the slope of the straight that explains the represented data can be calculated. The Hurst exponent may thus be determined in accordance with:

$$H_{R/S} = \frac{R(\tau)}{S(\tau)}$$

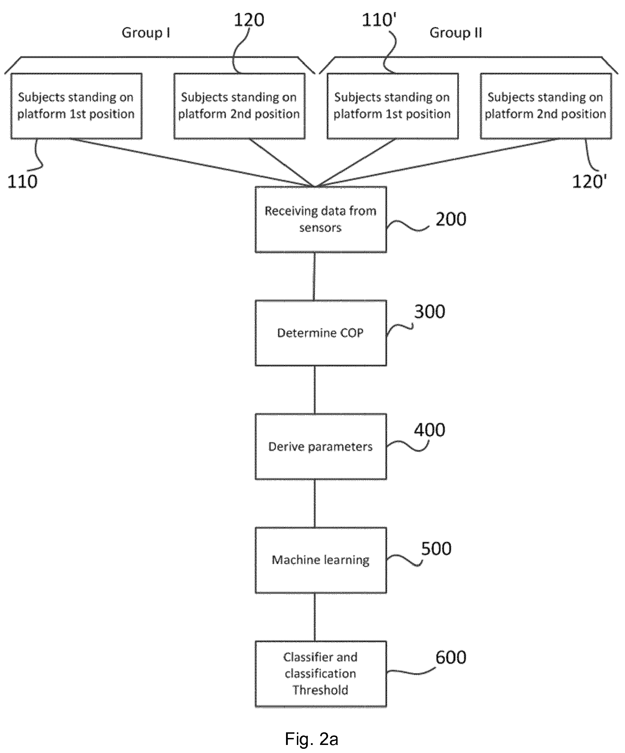

Figure 2a schematically illustrates an example of a method for determining a classifier and a classification threshold for use in a method for diagnosing HA.

A plurality of subjects may be divided into two groups, Group I and Group II, having different degrees of HA. For example, Group I may suffer from HA, whereas group II does not.

Each of the subjects may stand 110 (and 110') on a pressure platform during a predetermined test period in a first unilateral stance and in a second unilateral stance 120 (and 120'). Inventors have found that particularly unilateral stances are able to give information regarding a degree of HA. Further it is beneficial if unilateral stances on preferred legs are compared and that unilateral stances on non-preferred legs are compared. More information can be extracted from such comparisons than from comparisons of unilateral stances on right legs and left legs.

Alternatively, a unilateral stance on a preferred leg may be combined e.g. with a bilateral stance with eyes open and/or a bilateral stance with eyes closed.

The predetermined time periods may be between 10 - 45 seconds long. Longer test periods may be hard to endure for subjects and shorter time periods may not give sufficient information to be able to obtain a classifier.

During the test periods, data may be received 200 from the sensors of the pressure platform. The position of the COP may be determined 300 at any given moment during the test period. And various of the parameters explained before may be calculated 400 for each of the subjects.

Inventors have found that particularly, parameters indicative of a velocity of the COP, a shape and size of an area covered by the COP during the test period and parameters of a power spectral density (PSD) can be indicative of HA. At least one or more parameters indicative of velocity, shape and size of the area and the PSD may be determined for each of the subjects of group I and group II. These parameters may then be used in a process of machine learning.

Many of the parameters may be determined for RD ("resultant distance"; i.e. total displacement), AP (along the antero-posterios axis) and ML (transverse axis. The parameters may thus be organized with respect to position ("preferred" or "non-preferred"), and axis (RD, AP, or ML).

For each of the subjects, the degree of HA is known, so that processes like quadratic discriminant analysis or vectorial support machine models can be used 500 for determining an identifier and a corresponding classification threshold 600. The classifier and classification threshold may then be used for any subject (for which the degree of HA is not known) to indicate with sufficient reliability whether the person suffers from HA or not, based only the unilateral stances on a pressure platform. No further tests such as X-rays would be necessary.

In this example, a classifier and classification threshold were determined for distinguishing between suffering from HA or not. In a very similar manner, classifiers and classification thresholds could be determined for distinguishing e.g. between moderate-normal-severe HA. In this case, three groups of subjects need to be tested for which the degree of HA is known.

Test periods involving unilateral stances may also be complemented with bilateral stances for the subjects for which the degree of HA is known in order to improve the reliability of classifiers and classification thresholds. Inventors have found that a unilateral stance may convey more information regarding the presence and extent of HA than bilateral stances. However, inventors have also found that specific parameters of bilateral stances with eyes closed or eyes open can also serve to derive an identifier and a classification threshold, e.g. parameters indicative of PSD.

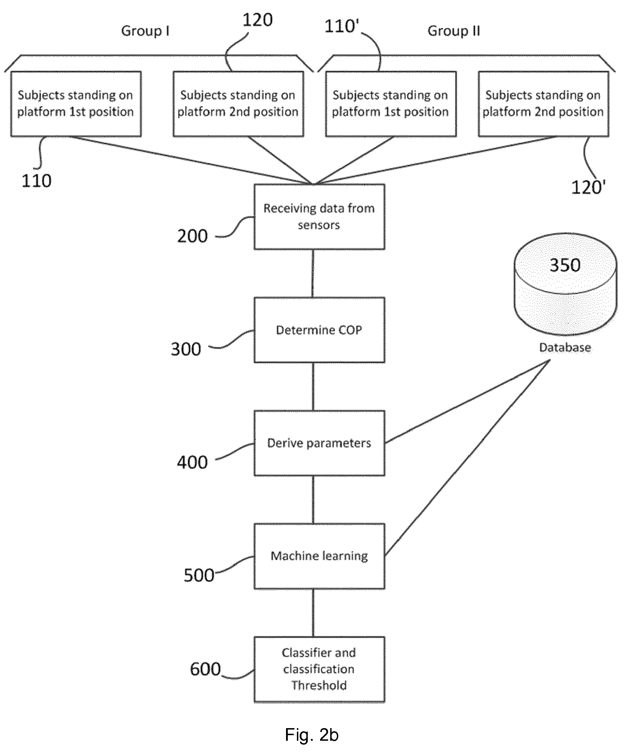

Figure 2b schematically illustrates an alternative example of a method for determining a classifier and a classification threshold. Figure 2b merely serves to illustrate that the data from groups I and II need not necessarily be fresh data. Many data may already be available from one or more databases 350, since using a pressure platform was already a known diagnostic instrument for determining (un)balance in patients. It is only with the present invention that methods and systems are provided which are able to provide classifiers, classification thresholds and diagnosis.

From previously existing data, whenever the degree of HA was known (or subsequently determined) and stored for a patient, the parameters derived from the stabilogram could be used in a process for deriving a classifier and a classification threshold.

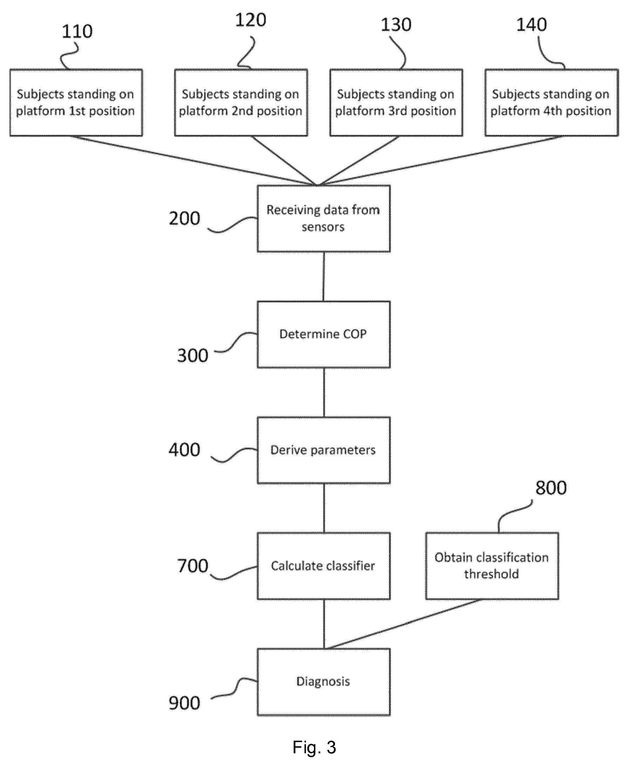

Figure 3 schematically illustrates an example of a method for diagnosing HA according to an implementation. In this particular example, it may be assumed that in a previous stage, a classifier and one or more classification thresholds have been determined at block 800. In this example, it may further be assumed that in the determination of the classifier, data has been gathered from both unilateral stances and bilateral stances. It will be clear from previous explanations that in other examples, only unilateral stances need to be taken into account, or in yet a further example, the unilateral stances and one of the bilateral stances (eyes open or eyes closed) may be taken into account.

A subject may thus perform the test for four different positions 110, 120, 130, 140, and during these tests, the data from the pressure sensors may be sent to a computing system for its processing 200. The movements of the COP during each of these tests may be determined at block 300 and the relevant parameters (those parameters that are to be taken into account for the classifier) can be determined at block 400 substantially in real-time.

The classifier may thus be calculated at block 700. From a comparison between the value of the classifier and the one or more previously obtained classification thresholds, a diagnosis may be derived. Depending on the classification threshold used, the diagnosis at block 900 may be e.g. HA yes or no, moderate or severe case of HA, HA proliferate in knees or rather in ankles. In further examples, various identifiers may be used, wherein e.g. a first identifier is calculated to determine the severity of HA and second identifier is used to indicate the joint most affected. At block 900, apart from the diagnosis a proposed dosage regime of a medicine may be output as well.

In an experiment developed by the inventors, a classifier was based on 10 parameters, notably:

| Parameter | Axis | Stance |
|---|---|---|
| RMS | AP | Unilateral preferred leg |
| Average velocity | AP | Unilateral preferred leg |
| Mean frequency | RD | Bilateral eyes closed |
| Mean frequency | RD | Unilateral preferred leg |
| Fractal dimension taking as metric space a circle with diameter equal to range | RD | Unilateral preferred leg |
| Fractal dimension taking as metric space a circle with a radius such that 95% of COP is enclosed | AP | Unilateral preferred leg |
| PSD power at frequencies between 0.015 - 0.5 Hz | RD | Bilateral eyes open |
| Median frequency | RD | Bilateral eyes open |
| 95% frequency | AP | Bilateral eyes closed |
| Critical point | RD | Bilateral eyes open |

A vector X may be formed by these 10 parameters. And a resultant classifier may be obtained from:

$$K + X.L + X.Q.X'$$

The constants K, L and Q may result from a quadratic discriminant analysis. If $K + X.L + X.Q.X' > 0$, then no HA. If $K + X.L + X.Q.X' \leq 0$ then HA.

Inventors found that using methods as hereinbefore described it was possible to derive classifiers and classification thresholds having a reliability of more than 90% when used for diagnosing HA.

For reasons of completeness, various aspects of the present disclosure are set out in the following numbered clauses:

Clause 1. Method for determining a classifier and a classification threshold for use in a method for diagnosing haemophilic arthropathy comprising

(i1) a plurality of subjects standing on a pressure platform in a first position during at least a first test period, the pressure platform having a plurality of pressure sensors for measuring a pressure locally;
(ii) receiving data from the pressure sensors indicative of local pressures during the test periods;
(iii) based on the received data from the pressure sensors determining a location of a centre of pressure during the test periods; and
(iv) based on the location of the centre of pressure during the test period deriving

(iv1) one or more parameters indicative of a velocity of the centre of pressure during the test periods,
(iv2) one or more parameters indicative of a shape of an area covered by the centre of pressure during the test periods,
(iv3) one or more parameters indicative of a size of the area covered by the centre of pressure during the test periods,
the plurality of subjects comprises a first group of subjects and a second group of subjects, wherein the first group of subjects suffer to a different extent from haemophilic arthropathy than the second group of subjects, the first position is a unilateral stance on a preferred leg of the subjects, and wherein
(v) the classifier and the classification threshold are determined in a process of machine learning based on the derived parameters for the first and second groups of subjects.

Clause 2. Method for determining a classifier according to clause 1, wherein step (v) comprises a quadratic discriminant analysis or a vectorial support machine model of the derived parameters.

Clause 3. Method according to clause 1 or 2, wherein the method further comprises

(i2) the plurality of subjects standing on the pressure platform in a second position during a second test period, and wherein
the second position is a unilateral stance on the non-preferred leg of the subjects.

Clause 4. Method according to any of clauses 1 - 3, wherein the method further comprises

(i3) the plurality of subjects standing on the pressure platform in a third position during a third test period and wherein
the third position is a bilateral stance with eyes closed.

Clause 5. Method according to any of clauses 1 - 4, wherein the method further comprises

(i4) the plurality of subjects standing on the pressure platform in a fourth position during a fourth test period, and wherein
the fourth position is a bilateral stance with eyes open.

Clause 6. Method according to any of clauses 1 - 5, further comprising based on the location of the centre of pressure during the test period deriving (iv4) one or more parameters of a power spectral density of the centre of pressure during the test periods.

Clause 7. Method according to clause 6, wherein one or more parameters of a power spectral density of the centre of pressure include one or more of the following:

- a total power of the power spectral density,
- a power in low frequencies from 0.015 to 0.5 Hz,
- a power in median frequencies from 0.5 to 2 Hz,
- a power in high frequencies from 2 to 6 Hz,
- a median power frequency being equal to a frequency below which 50% of the total power lies,
- a 95% power frequency being equal to a frequency below which 95% of the total power lies,
- a centroidal frequency being a frequency at which the spectral mass is concentrated, and
- a frequency dispersion, the frequency dispersion being a measure of a variability of the frequency content of the power spectral density.

Clause 8. Method according to any of clauses 1 - 7, wherein the method further comprises prior to step (v) selecting from the derived parameters a selection of parameters having relatively small prediction errors based on forward feature selection and/or backward feature selection algorithms, and wherein

(v) the classifier and the classification threshold are determined in a process of machine learning based on the selection of parameters.

Clause 9. Method according to any of clauses 1 - 8, the method comprising making a pre-selection based on individual predictive values of the derived parameters prior to selecting from the derived parameters a selection of parameters having relatively small prediction errors.

Clause 10. Method according to any of clauses 1 - 9, wherein
the one or more parameters indicative of a shape of an area covered by the centre of pressure include one or more fractal dimensions indicative of the degree to which the position of the centre of pressure fills a metric space which it encompasses, including

- a fractal dimension wherein the metric space is a circle having a diameter which is equal to the range of the centre of pressure, and/or
- a fractal dimension wherein the metric space is a circle having a radius such that the centre of pressure is within the circle 95% of the time and/or
- a fractal dimension wherein the metric space is an ellipse such that the centre of pressure is within the ellipse 95% of the time.

Clause 11. Method according to any of clauses 1 - 10, wherein
the one or more parameters indicative of the size of the area covered by the centre of pressure, include one or more of the following:

- a root mean square of the positions of the centre of pressure;
- an area of a circle encompassing the centre of pressure 95% of the time;
- an area of an ellipse encompassing the centre of pressure 95% of the time.

Clause 12. Method according to any of clauses 1 - 11, wherein
the one or more parameters indicative of a velocity of the centre of pressure include one or more of the following:

- a mean frequency determined as a number of revolutions per second of the centre of pressure if the centre of pressure had travelled the total distance travelled around a circle having a radius equal to the mean distance travelled
- an average velocity of the centre of pressure
- a sum of areas of triangles formed by two consecutive positions of the centre of pressure and an average centre of pressure.

Clause 13. Method according to any of clauses 1 - 12, wherein step (iv) furthermore comprises deriving one or more of the following:

(iv5) one or more parameters indicative of a total displacement of the centre of pressure
(iv6) one or more parameters derived from a stabilogram diffusion analysis and/or an R/S analysis.

Clause 14. Method according to any of clauses 1 - 13, wherein step (iv) comprises determining the location of the centre of pressure along an anteroposterior axis and along a sideways axis perpendicular to the anteroposterior axis, and determining a resultant positional vector of the resultant positional vector by summing the centre of pressure along the anteroposterior axis with the centre of pressure along the sideways axis, and wherein the derived parameters in step (v) comprise parameters derived from the location of the centre of pressure along the anteroposterior axis and parameters derived from the resultant positional vector of the centre of pressure.

Clause 15. Method according to any of clauses 1 - 14, wherein the test periods are between 10 - 45 seconds long, optionally between 15 - 35 seconds long.

Clause 16. Method according to any of clauses 1 - 15, wherein the classification threshold is a single value.

Clause 17. Method according to any of clauses 1 - 16, wherein the classification threshold is a function.

Clause 18. Method according to any of clauses 1 - 17, wherein the first group of subjects does not suffer from haemophilic arthropathy, and the second group of subjects suffers from haemophilic arthropathy.

Clause 19. Method according to any of clauses 1 - 18, wherein both the first and the second groups of subjects suffers from haemophilic arthropathy.

Clause 20. Method according to clause 19, wherein the first group of subjects has a more severe degree of haemophilic arthropathy than the second group of subjects.

Clause 21. Method according to clause 19, wherein in the first group of subjects a first joint is most affected, and wherein in the second group of subjects another joint is most affected.

Clause 22. Method for diagnosing haemophilic arthopathy comprising

(a1) a subject standing on a pressure platform in a first position during at least a first test period, the pressure platform having a plurality of pressure sensors for measuring a pressure locally;
(b) receiving data from the pressure sensors indicative of local pressures during the test periods;
(c) based on the received data from the pressure sensors determining a location of a centre of pressure during the test periods; and
(d) based on the location of the centre of pressure during the test periods derive

(d1) one or more parameters indicative of a velocity of the centre of pressure during the test periods,
(d2) one or more parameters indicative of a shape of an area covered by the centre of pressure during the test periods,
(d3) one or more parameters indicative of a size of the area covered by the centre of pressure during the test periods;

(e) calculating a value of a classifier previously derived by a method according to any of clauses 1 - 21; and
(f) determining the extent of haemophilic arthropathy of the subject by comparing the value of the classifier with a classification threshold previously derived by a method according to any of clauses 1 - 21, and wherein the first position is a unilateral stance on a preferred leg of the subject.

Clause 23. Method according to clause 22, wherein the method further comprises

(i2) the plurality of subjects standing on the pressure platform in a second position during a second test period, and wherein
the second position is a unilateral stance on the non-preferred leg of the subjects.

Clause 24. Method according to clause 22 or 23, wherein the method further comprises

(a3) the plurality of subjects standing on the pressure platform in a third position during a third test period and wherein
the third position is a bilateral stance with eyes closed.

Clause 25. Method according to any of clauses 22 - 24, wherein the method further comprises

(a4) the plurality of subjects standing on the pressure platform in a fourth position during a fourth test period and wherein
the fourth position is a bilateral stance with eyes open.

Clause 26. Method according to any of clauses 22 - 25, further comprising based on the location of the centre of pressure during the test period deriving

(iv4) one or more parameters of a power spectral density of the centre of pressure during the test periods.

Clause 27. Method according to clause 26, wherein one or more parameters of a power spectral density of the centre of pressure include one or more of the following:

- a total power of the power spectral density,
- a power in low frequencies from 0.015 to 0.5 Hz,
- a power in median frequencies from 0.5 to 2 Hz,
- a power in high frequencies from 2 to 6 Hz,
- a median power frequency being equal to a frequency below which 50% of the total power lies,
- a 95% power frequency being equal to a frequency below which 95% of the total power lies,

- a centroidal frequency being a frequency at which the spectral mass is concentrated, and
- a frequency dispersion, the frequency dispersion being a measure of a variability of the frequency content of the power spectral density.

Clause 28. Method according to any of clauses 22 - 27, wherein the steps (b) - (f) are implemented in a computing apparatus.

Clause 29. Method according to any of clauses 22 - 28, wherein
the one or more parameters indicative of a shape of an area covered by the centre of pressure include one or more fractal dimensions indicative of the degree to which the position of the centre of pressure fills a metric space which it encompasses, including

- a fractal dimension wherein the metric space is a circle having a diameter which is equal to the range of the centre of pressure, and/or
- a fractal dimension wherein the metric space is a circle having a radius such that the centre of pressure is within the circle 95% of the time and/or
- a fractal dimension wherein the metric space is an ellipse such that the centre of pressure is within the ellipse 95% of the time.

Clause 30. Method according to any of clauses 22 - 29, wherein
the one or more parameters indicative of the size of the area covered by the centre of pressure, include one or more of the following:

- a root mean square of the positions of the centre of pressure;
- an area of a circle encompassing the centre of pressure 95% of the time;
- an area of an ellipse encompassing the centre of pressure 95% of the time.

Clause 31. Method according to any of clauses 22 - 30, wherein
the one or more parameters indicative of a velocity of the centre of pressure include one or more of the following:

- a mean frequency determined as a number of revolutions per second of the centre of pressure if the centre of pressure had travelled the total distance travelled around a circle having a radius equal to the mean distance travelled;
- an average velocity of the centre of pressure;
- a sum of areas of triangles formed by two consecutive positions of the centre of pressure and an average centre of pressure.

Clause 32. Method according to any of clauses 22 - 31, wherein step (d) comprises determining the location of the centre of pressure along an anteroposterior axis and along a sideways axis perpendicular to the anteroposterior axis, and determining a resultant positional vector of the resultant positional vector by summing the centre of pressure along the anteroposterior axis with the centre of pressure along the sideways axis, and wherein the derived parameters in step (e) comprise parameters derived from the location of the centre of pressure along the anteroposterior axis and parameters derived from the resultant positional vector of the centre of pressure.

Clause 33. Method according to any of clauses 22 - 32, wherein the test periods are between approximately 10 - 40 seconds long, optionally between 15 - 35 seconds long.

Clause 34. Computer program comprising program instructions for causing a computer to perform the steps (b) - (f) of any of the clauses 22 - 33.

Clause 35. Computer program product comprising a computer program according to clause 34 embodied on a storage medium.

Clause 36. Computer program product comprising a computer program according to clause 34 carried on a carrier signal.

Clause 37. Computing system adapted to be connected to a pressure platform having a plurality of sensors and programmed to perform any of the methods according to any of clauses 22 - 33.

Clause 38. System for performing a method according to any of clauses 22 - 33 comprising a pressure platform having a plurality of pressure sensors and a computing system according to clause 37.

Although only a number of particular embodiments and examples of the invention have been disclosed herein, it will be understood by those skilled in the art that other alternative embodiments and/or uses of the invention and obvious modifications and equivalents thereof are possible. Furthermore, the present invention covers all possible combinations of the particular embodiments described. Thus, the scope of the present invention should not be limited by particular embodiments, but should be determined only by a fair reading of the claims that follow.

**Claims**

1. Method for determining a classifier and a classification threshold for use in a method for diagnosing haemophilic arthropathy comprising

   (i1) a plurality of subjects standing on a pressure platform in a first position during at least a first test period, the pressure platform having a plurality of pressure sensors for measuring a pressure locally;
   (ii) receiving data from the pressure sensors indicative of local pressures during the test periods;
   (iii) based on the received data from the pressure sensors determining a location of a centre of pressure during the test periods; and
   (iv) based on the location of the centre of pressure during the test periods deriving
   (iv1) one or more parameters indicative of a velocity of the centre of pressure during at least one of the test periods,
   (iv2) one or more parameters indicative of a shape of an area covered by the centre of pressure during at least one of the test periods,
   (iv3) one or more parameters indicative of a size of the area covered by the centre of pressure during at least one of the test periods; wherein

   the plurality of subjects comprises a first group of subjects and a second group of subjects, wherein the first group of subjects suffer to a different extent from haemophilic arthropathy than the second group of subjects, wherein
   the first position is a unilateral stance on a preferred leg of the subjects, and wherein

   (v) the classifier and the classification threshold are determined in a process of machine learning based on the derived parameters for the first and second groups of subjects.

2. Method for determining a classifier according to claim 1, wherein step (v) comprises a quadratic discriminant analysis or a vectorial support machine model of the derived parameters.

3. Method according to claim 1 or 2, wherein
   the one or more parameters indicative of a shape of an area covered by the centre of pressure include one or more fractal dimensions indicative of the degree to which the position of the centre of pressure fills a metric space which it encompasses, including

   - a fractal dimension wherein the metric space is a circle having a diameter which is equal to the range of the centre of pressure, and/or
   - a fractal dimension wherein the metric space is a circle having a radius such that the centre of pressure is within the circle 95% of the time and/or
   - a fractal dimension wherein the metric space is an ellipse such that the centre of pressure is within the ellipse 95% of the time.

4. Method according to any of claims 1 - 3, wherein
   the one or more parameters indicative of the size of the area covered by the centre of pressure, include one or more of the following:

   - an area of a circle encompassing the centre of pressure 95% of the time;
   - a root mean square of the positions of the centre of pressure;
   - an area of an ellipse encompassing the centre of pressure 95% of the time.

**5.** Method according to any of claims 1 - 4, wherein
the one or more parameters indicative of a velocity of the centre of pressure include one or more of the following:

- a mean frequency determined as a number of revolutions per second of the centre of pressure if the centre of pressure had travelled the total distance travelled around a circle having a radius equal to the mean distance travelled
- an average velocity of the centre of pressure
- a sum of areas of triangles formed by two consecutive positions of the centre of pressure and an average centre of pressure.

**6.** Method according to any of claims 1 - 5, wherein the method further comprises

(i2) the plurality of subjects standing on the pressure platform in a second position during a second test period, and wherein the second position is a unilateral stance on the non-preferred leg of the subjects and/or comprises
(i3) the plurality of subjects standing on the pressure platform in a third position during a third test period and wherein the third position is a bilateral stance with eyes closed
and/or comprises
(i4) the plurality of subjects standing on the pressure platform in a fourth position during a fourth test period, and wherein the fourth position is a bilateral stance with eyes open.

**7.** Method according to any of claims 1 - 6, further comprising based on the location of the centre of pressure during the test period deriving (iv4) one or more parameters of a power spectral density of the centre of pressure during the test periods.

**8.** Method according to any of claims 1 - 7, wherein step (iv) comprises determining the location of the centre of pressure along an anteroposterior axis and along a sideways axis perpendicular to the anteroposterior axis, and determining a resultant positional vector of the resultant positional vector by summing the centre of pressure along the anteroposterior axis with the centre of pressure along the sideways axis, and
wherein the derived parameters in step (v) comprise parameters derived from the location of the centre of pressure along the anteroposterior axis and parameters derived from the resultant positional vector of the centre of pressure.

**9.** Method according to any of claims 1 - 8, wherein the test periods are between 10 - 45 seconds long, optionally between 15 - 35 seconds long.

**10.** Method according to any of claims 1 - 9, wherein the first group of subjects does not suffer from haemophilic arthropathy, and the second group of subjects suffers from haemophilic arthropathy.

**11.** Method for diagnosing haemophilic arthopathy comprising

(a1) a subject standing on a pressure platform in a first position during a first test period, the pressure platform having a plurality of pressure sensors for measuring a pressure locally;
(b) receiving data from the pressure sensors indicative of local pressures during the test periods;
(c) based on the received data from the pressure sensors determining a location of a centre of pressure during the test periods; and
(d) based on the location of the centre of pressure during the test periods derive

(d1) one or more parameters indicative of a velocity of the centre of pressure during the test periods,
(d2) one or more parameters indicative of a shape of an area covered by the centre of pressure during the test periods,
(d3) one or more parameters indicative of a size of the area covered by the centre of pressure during the test periods,

(e) calculating a value of a classifier previously derived by a method according to any of claims 1 - 10; and
(f) determining the extent of haemophilic arthropathy of the subject by comparing the value of the classifier with a classification threshold previously derived by a method according to any of claims 1 - 10, and wherein
the first position is a unilateral stance on a preferred leg of the subject.

**12.** Method according to claim 11, wherein the steps (b) - (f) are implemented in a computing apparatus.

13. Computer program comprising program instructions for causing a computer to perform the steps (b) - (f) of -claim 11 or 12.

14. Computer program product according to claim 28 embodied on a storage medium or carried on a carrier signal.

15. Computing system adapted to be connected to a pressure platform having a plurality of sensors and programmed to perform any of the methods according to claim 11 or 12.

Fig. 1a

COP
AP axis

COP
sideways axis

Fig. 1b

Group I

120

110'

Group II

Subjects standing on platform 1st position

Subjects standing on platform 2nd position

Subjects standing on platform 1st position

Subjects standing on platform 2nd position

110

120'

Receiving data from sensors — 200

Determine COP — 300

Derive parameters — 400

Machine learning — 500

Classifier and classification Threshold — 600

Fig. 2a

EP 3 171 286 A1

Fig. 2b

110

Subjects standing on platform 1st position

120

Subjects standing on platform 2nd position

130

Subjects standing on platform 3rd position

140

Subjects standing on platform 4th position

200

Receiving data from sensors

300

Determine COP

400

Derive parameters

700

Calculate classifier

800

Obtain classification threshold

900

Diagnosis

Fig. 3

Europäisches
Patentamt

European
Patent Office

Office européen
des brevets

# EUROPEAN SEARCH REPORT

Application Number

EP 15 38 2570

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | US 2012/029345 A1 (MAHFOUZ MOHAMED R [US] ET AL) 2 February 2012 (2012-02-02)<br>* paragraph [0057] - paragraph [0061] *<br>* paragraph [0109] - paragraph [0114] *<br>* figures 9, 10, 28, 29, 33, 34 *<br>* claim 26 *<br>----- | 1-10<br><br>11-15 | INV.<br>G06F19/00 |

TECHNICAL FIELDS
SEARCHED      (IPC)

G06F

The present search report has been drawn up for all claims

| Place of search | Date of completion of the search | Examiner |
|---|---|---|
| Munich | 30 May 2016 | Wittke, Claudia |

CATEGORY OF CITED DOCUMENTS

X : particularly relevant if taken alone
Y : particularly relevant if combined with another
　　document of the same category
A : technological background
O : non-written disclosure
P : intermediate document

T : theory or principle underlying the invention
E : earlier patent document, but published on, or
　　after the filing date
D : document cited in the application
L : document cited for other reasons
............................................................
& : member of the same patent family, corresponding
　　document

EPO FORM 1503 03.82 (P04C01)

**EP 3 171 286 A1**

## ANNEX TO THE EUROPEAN SEARCH REPORT
## ON EUROPEAN PATENT APPLICATION NO.

EP 15 38 2570

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2016

| Patent document cited in search report | Publication date | Patent family member(s) | Publication date |
|---|---|---|---|
| US 2012029345 A1 | 02-02-2012 | NONE | |

EPO FORM P0459

For more details about this annex : see Official Journal of the European Patent Office, No. 12/82